# DEMANDE DE BREVET EUROPEEN

(11) **EP 1 782 790 A1**
(43) Date de publication de la demande: **09.05.2007**
(21) Numéro de dépôt: 06122984.5
(22) Date de dépôt: 26.10.2006
(51) Int. Cl.: A61K 8/37, A61K 8/73, A61Q 3/02

(54) **Vernis à ongles comprenant un ester de cellulose et des solvants esters courts en un ratio défini**

(30) Priorité: 04.11.2005 FR 0553350
(71) Demandeur: L'Oreal, 75008 Paris (FR)
(72) Inventeur: Francois, Nathalie, 91000, Evry (FR); Cavazzuti, Roberto, 75015, Paris (FR); Coffey-Dawe, Lizabeth-Anne, 93600, Aulnay Sous Bois (FR)
(74) Mandataire: Duvert, Sandra

(57) **Abrégé**

La présente invention a pour objet un vernis à ongles comprenant un ester de cellulose et un milieu solvant organique comprenant de l'acétate d'éthyle et de l'acétate de butyle en un ratio acétate d'éthyle/acétate de butyle allant de 87/25 à 100/1.

La présente invention a également pour l'utilisation d'un tel vernis pour obtenir un film sur les ongles qui sèche rapidement, qui est brillant, de bonne tenue et résistant à l'eau.

## Description

La présente invention a trait à un vernis à ongles comprenant un milieu solvant organique comprenant au moins deux solvants en un ratio spécifique et un ester de cellulose.
La composition de vernis à ongles, colorée ou transparente, peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles.

D'une manière générale, les vernis à ongles comprennent au moins un agent filmogène, un agent plastifiant, un agent gélifiant et un milieu solvant qui peut être aqueux ou organique.
Le solvant peut être un alcool tel que l'éthanol, l'isopropanol ou le butanol, un acétate tel que l'acétate d'éthyle ou de butyle, une cétone.
Leur utilisation consiste à appliquer une ou plusieurs couches dudit vernis sur la surface de l'ongle, puis à laisser évaporer le solvant de manière à déposer un film sur l'ongle.
Les couches de vernis à ongles déposées présentent toutefois, juste après leur application, des risques de blessures par friction et/ou accrochage sur les corps étrangers. Aussi, il est souhaitable que ces vernis sèchent rapidement après avoir été appliqués.
Dans l'art antérieur, la vitesse de séchage est optimisée en sélectionnant les composants volatils, c'est à dire le solvant et éventuellement le diluant, entrant dans la composition du vernis à ongles, selon leur nature chimique et leur volatilité.
Cette optimisation a pour contrainte une limitation de la formulation du vernis à ongles, car les composants trop volatils peuvent pénaliser la qualité du maquillage obtenu. En effet, des défauts peuvent apparaître lors de l'application du vernis à ongles : formation de stries sur la couche de vernis, séchage trop rapide du pinceau lors de l'application. On obtient un film non homogène et pas suffisamment lisse; il est peu brillant et présente une tenue non satisfaisante.

La demanderesse a constaté que l'utilisation d'un ester de cellulose et d'acétate d'éthyle en proportion majoritaire dans le milieu solvant d'un vernis à ongles permet d'obtenir, après application sur les ongles, un film qui sèche rapidement tout en étant homogène et lisse; et présentant de bonne propriétés de tenue et de brillance.

La présente invention a donc pour objet un vernis à ongles comprenant un ester de cellulose et un milieu solvant organique comprenant de l'acétate d'éthyle et de l'acétate de butyle en un ratio acétate d'éthyle/acétate de butyle allant de 87/25 à 100/1, de préférence de 88/24 à 10/1, et mieux de 88/24 à 6/1, l'acétate d'éthyle étant présent en une teneur supérieure ou égale à 25% en poids par rapport au poids total de la composition et ledit vernis comprenant moins de 5% en poids d'acétone.

L'invention a encore pour objet un procédé cosmétique de maquillage ou de soin non thérapeutique des ongles comprenant l'application sur les ongles d'au moins une couche d'une composition de vernis à ongles telle que définie ci-dessus.

L'invention a également pour objet l'utilisation d'une composition de vernis à ongles comprenant au moins un ester de cellulose et un milieu solvant organique comprenant de l'acétate d'éthyle et de l'acétate de butyle en un ratio acétate d'éthyle/acétate de butyle allant de 87/25 à 100/1, l'acétate d'éthyle étant présent en une teneur supérieure ou égale à 25% en poids par rapport au poids total de la composition et ledit vernis comprenant moins de 5% en poids d'acétone, pour obtenir, après dépôt sur l'ongle, un film qui sèche rapidement, qui est brillant, de bonne tenue et résistant à l'eau.

Le vernis à ongles comprend un milieu cosmétiquement acceptable, c'est-à-dire un milieu non toxique et susceptible d'être appliqué sur les matières kératiniques d'êtres humains, en particulier les ongles.

### Milieu solvant organique

Le milieu solvant organique de la composition cosmétique comprend de l'acétate d'éthyle et de l'acétate de butyle en un ratio acétate d'éthyle/acétate de butyle allant de 87/25 à 100/1, de préférence de 88/24 à 10/1, et mieux de 88/24 à 6/1.

L'acétate d'éthyle peut être présent en une teneur allant de 25 à 70% en poids par rapport au poids total de la composition, de préférence de 30 à 60% en poids et mieux de 35 à 50% en poids.
L'acétate de butyle peut être présent en une teneur allant de 1 à 30% en poids par rapport au poids total de la composition, de préférence de 5 à 20% en poids.
L'acétate d'éthyle est présent dans la composition selon l'invention en une teneur supérieure ou égale à 25% en poids par rapport au poids total de la composition, de préférence supérieure ou égale à 30% en poids et mieux, supérieure ou égale à 35% en poids.

Selon un mode de réalisation, la composition comprend de l'acétate de butyle en une teneur inférieure ou égale à 30% en poids par rapport au poids total de la composition, de préférence inférieure ou égale à 25% en poids, de préférence encore inférieure ou égale à 20% en poids, et mieux inférieure ou égale à 15% en poids.

Le milieu solvant organique de la composition peut comprendre, outre l'acétate d'éthyle et l'acétate de butyle, un solvant organique dit additionnel ou un mélange de solvants organiques additionnels.

Le solvant organique additionnel peut être choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) autres que l'acétate d'éthyle et l'acétate de butyle, tels que l'acétate de méthyle, l'acétate de propyle, l'acétate d'isopropyle, , l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

De préférence, l'acétate d'éthyle et l'acétate de butyle sont les solvants majoritaires en poids par rapport au poids total des solvants organiques du milieu solvant organique de la composition.

Le vernis à ongles selon l'invention comprend moins de 5% d'acétone, de préférence mois de 3% et mieux, est totalement exempt d'acétone.

Le milieu solvant organique peut représenter de 10 à 95% en poids, par rapport au poids total de la composition, de préférence de 15% à 80% en poids, et mieux de 20 à 70% en poids.

Le milieu cosmétiquement acceptable de la composition selon l'invention peut éventuellement comprendre un milieu aqueux.
La teneur en milieu aqueux de la composition peut aller de 5 à 95 % en poids par rapport au poids total de la composition, de préférence de 50 à 70% en poids.

### Polymère filmogène

La composition comprendre avantageusement un polymère filmogène choisi parmi les esters de cellulose
Selon la présente invention, on entend par "polymère filmogène", un polymère apte à former à lui seul ou en présence d'un agent auxiliaire de filmification, un film continu et adhérent sur un support, notamment sur les matières kératiniques.

Les esters de cellulose peuvent être choisis parmi les esters issus de la réaction d'une partie des fonctions hydroxyles libres de la cellulose avec un acide carboxylique ou un dérivé d'acide carboxylique comprenant de 1 à 10 atomes de carbone tels que les acétates, les propionates, les butyrates, les isobutyrates, les acétobutyrates, les acétopropionates de cellulose et leurs mélanges.

L'ester de cellulose peut être présent dans la composition selon l'invention en une teneur en matières sèches allant de 0,1 % à 20 % en poids, par rapport au poids total de la composition, de préférence allant de 0,5 % à 15 % en poids, et mieux de 1 % à 10 % en poids.

La composition comprend avantageusement au moins un polymère filmogène additionnel choisi parmi les polymères synthétiques, de type radicalaire ou de type polycondensat, les polymères d'origine naturelle et leurs mélanges.

Le polymère filmogène additionnel peut être choisi en particulier parmi les polymères cellulosiques tels que la nitrocellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.

Comme polymère filmogène additionnel, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre.
De tels polymères sont décrits par exemple dans les documents EP 1411069 ou WO04/028488.

Le polymère filmogène peut être présent dans la composition selon l'invention en une teneur en matières sèches allant de 0,1 % à 60 % en poids, par rapport au poids total de la composition, de préférence allant de 2 % à 40 % en poids, et mieux de 5 % à 25 % en poids.

### Agent auxiliaire de filmification

Pour améliorer les propriétés filmogènes de la composition de vernis à ongles un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- des dérivés oxyéthylénés, tels que les huiles oxyéthylénées, notamment les huiles végétales, telles que l'huile de ricin ; et
- leurs mélanges.

Le type et la quantité d'agent plastifiant et/ou de coalescence peuvent être choisis par l'homme du métier sur la base de ses connaissances générales.
Par exemple, la teneur en agent plastifiant et/ou de coalescence peut aller de 0,01 % à 20% et en particulier de 0,5 % à 10 % en poids par rapport au poids total de la composition.

### Agent épaississant

La composition peut comprendre un agent épaississant qui peut en particulier être choisi parmi : les silices hydrophobes telles que celles décrites dans le document EP-A-898960, et par exemple commercialisées sous les références "AEROSIL R812®" par la société Degussa, "CAB-O-SIL TS-530^{®}", "CAB-O-SIL TS-610^{®}", "CAB-O-SIL TS-720^{®}"par la société Cabot, "AEROSIL R972^{®}", "AEROSIL R974^{®}" par la société Degussa ; les silices hydrophiles telles que celle commercialisée sous la référence "AEROSIL 200^{®}" par la société Degussa, les argiles telles que la montmorillonite, les argiles modifiées telles que les bentones par exemple, l'hectorite stéaralkonium, la bentonite stéaralkonium , les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La proportion totale en agent(s) épaississant(s) dans les compositions selon l'invention peut aller de à 0,01 à 15 % en poids, par rapport au poids total de la composition, de préférence de 0,5 à 15% et mieux de 0,5 à 10% en poids.

### Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon@) (Orgasol® de chez Atochem), de poly-β-alanine et de polyéthylène, les poudres de polymères de tétrafluoroéthylène (Téflon@), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads@ de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent de manière non limitative l'invention. Sauf indication contraire, les quantités indiquées sont des pourcentages massiques.

L'invention est illustrée plus en détail dans les exemples suivants. Sauf indication contraire, les quantités sont données en pourcentage en poids par rapport au poids total de la composition.

### Exemples 1 à 5 : Vernis à ongles

On prépare des vernis à ongles ayant la composition suivante (% en poids) :

| | **Exemple 1** | **Exemple 2** | **Exemple 3** | **Exemple 4** | **Exemple 5** |
|---|---|---|---|---|---|
| Acétobutyrate de cellulose (CAB 381,05 de Eastman Chemical) | 2,976 | 2,976 | 2,976 | 2,976 | 2,976 |
| Nitrocellulose à 30 d'alcool isopropylique (viscosité: E22 - 1/2 S) | 10,35 | 10,44 | 10,6 | 8,84 | 9,54 |
| Nitrocellulose à 30 % d'alcool isopropylique (Azur E80 de Bergerac) | 1,68 | 1,64 | 1,7 | 1,92 | 2,84 |
| Résine N-éthyl O,P-toluènesulfonamide | 4,09 | 3,91 | 4 | 4,25 | 4,75 |
| Résine alkyde glycérophtalique ésterifiée par acides gras ramifiés⁽¹⁾ dans l'acétate d'éthyle à 70% | 13,40 | 13,47 | 13,5 | 11,9 | 13,56 |
| Alcool isopropylique | 3,1 | 3,11 | 3,13 | 2,52 | 3,23 |
| Hectorite modifiée stéaryl benzyl diméthyl ammonium (BENTONE 27 V d'Elementis) | 0,98 | 0,99 | 1 | 0,95 | - |
| Laque Red 7 | 0,0075 | 0,75 | 0,08 | - | - |
| Laque Yellow 5 | 0,01 | - | - | - | - |
| Laque Red 6 | 0,012 | - | - | - | - |
| Oxyde de titane traité oxyde de polyéthylène | 1,25 | 0,16 | 0,05 | - | - |
| Mica oxyde de titane | | | | 10 | - |
| Oxyde de fer noir | 0,002 | - | - | - | - |
| Laque Red 34 | - | - | 0,318 | - | - |
| Acétate d'éthyle | 47,06 | 46,34 | 46,9 | 45 | Qsp 100 |
| Acétyl citrate de tributyle | 2,71 | 2,92 | 2,9 | 1,94 | 2,11 |
| Acétate de butyle | 12,31 | 13,22 | 12,9 | 9,6 | - |
| Acide citrique, 1 H2O | 0,98 | 0,99 | 0,04 | 0,04 | - |

| | | | | | |
|---|---|---|---|---|---|
| ⁽¹⁾ commercialisé par SHELL sous le nom de marque CARDURA 30^{®}. | | | | | |

### Exemples 6 à 8 : Vernis à ongles

On prépare un vernis à ongles selon l'invention (exemple 6 et 7) comprenant un ester de cellulose (acétobutyrate de cellulose) et de l'acétate d'éthyle et de l'acétate de butyle en un acétate d'éthyle/acétate de butyle d'environ 3,9 et un vernis hors invention (exemple 8) ne comprenant pas d'ester de cellulose.

| | **Exemple 6** | **Exemple 7** | **Exemple 8** |
|---|---|---|---|
| Acétobutyrate de cellulose (CAB 381,05 de Eastman Chemical) | 8,17 | 4,08 | - |
| Nitrocellulose à 30 d'alcool isopropylique (viscosité: E22 - 1/2 S) | 2,9 | 7,64 | 12,38 |
| Nitrocellulose à 30 % d'alcool isopropylique (Azur E80 de Bergerac) | - | 1,09 | 2,18 |
| Résine N-éthyl O,P-toluènesulfonamide | 5,07 | 5,07 | 5,07 |
| Résine alkyde glycérophtalique ésterifiée par acides gras ramifiés⁽¹⁾ dans l'acétate d'éthyle à 70% | 16,14 | 16,14 | 16,14 |
| Alcool isopropylique | 7,25 | 5,5 | 3,75 |
| Hectorite modifiée stéaryl benzyl diméthyl ammonium (BENTONE 27 V d'Elementis) | 1,15 | 1,15 | |
| Acétate d'éthyle | 45,78 | 45,78 | 45,78 |
| Acétyl citrate de tributyle | 1,78 | 1,78 | 1,78 |
| Acétate de butyle | 11,68 | 11,68 | 11,68 |
| Acide citrique, 1 H2O | 0,04 | 0,04 | 0,04 |

| | | | |
|---|---|---|---|
| ⁽¹⁾ commercialisé par SHELL sous le nom de marque CARDURA 30^{®}. | | | |

Les vernis des exemples 6 et 7 (selon l'invention), après application sur les ongles, ont été jugés comme séchant plus rapidement que celui de l'exemple 8 (hors invention).

## Revendications

1. Vernis à ongles comprenant un ester de cellulose et un milieu solvant organique comprenant de l'acétate d'éthyle et de l'acétate de butyle en un ratio acétate d'éthyle/acétate de butyle allant de 87/25 à 100/1, l'acétate d'éthyle étant présent en une teneur supérieure ou égale à 25% en poids par rapport au poids total de la composition et ledit vernis comprenant moins de 5% en poids d'acétone.

2. Vernis à ongles selon la revendication 1, **caractérisé en ce que** le ratio acétate d'éthyle/acétate de butyle va de 88/24 à 10/1, et mieux de 88/24 à 6/1.

3. Vernis à ongles selon la revendication 1 ou 2, **caractérisé en ce que** l'acétate d'éthyle est présent en une teneur allant de 25 à 70% en poids par rapport au poids total de la composition, de préférence de 30 à 60% en poids et mieux de 35 à 50% en poids.

4. Vernis à ongles selon l'une des revendications précédentes, **caractérisé en ce que** l'acétate d'éthyle est présent en une teneur supérieure ou égale à 30% en poids en poids par rapport au poids total de la composition et mieux, supérieure ou égale à 35% en poids.

5. Vernis à ongles selon l'une des revendications précédentes, **caractérisé en ce que** l'acétate de butyle est présent en une teneur allant de 1 à 30% en poids par rapport au poids total de la composition, de préférence de 5 à 20% en poids.

6. Vernis à ongles selon l'une des revendications précédentes, **caractérisé en ce que** l'acétate de butyle est présente en une teneur inférieure ou égale à 30% en poids par rapport au poids total de la composition.

7. Vernis à ongles selon l'une des revendications précédentes, **caractérisé en ce que** l'ester de cellulose est choisi parmi les esters issus de la réaction d'une partie des fonctions hydroxyles libres de la cellulose avec un acide carboxylique ou un dérivé d'acide carboxylique comprenant de 1 à 4 atomes de carbone tels que les acétates, les propionates, les butyrates, les isobutyrates, les acétobutyrates, les acétopropionates de cellulose et leurs mélanges.

8. Vernis à ongles selon l'une des revendications précédentes, **caractérisé en ce que** l'ester de cellulose est choisi parmi les acétobutyrates de cellulose.

9. Vernis à ongles selon l'une des revendications précédentes, **caractérisé en ce que** l'ester de cellulose représente de 0,1 % à 20 % en poids, par rapport au poids total de la composition, de préférence de 0,5 % à 15 % en poids, et mieux de 1 % à 10 % en poids.

10. Vernis à ongles selon l'une quelconque des revendications précédentes, **caractérisée en ce qu'**il comprend solvant organique additionnel

11. Vernis à ongles selon l'une quelconque des revendications précédentes, **caractérisé en ce que** l'acétate d'éthyle et l'acétate de butyle sont les solvants majoritaires en poids par rapport au poids total des solvants organiques du milieu solvant organique de la composition.

12. Vernis à ongles selon l'une quelconque des revendications précédentes, **caractérisé en ce qu'**il comprend un milieu aqueux.

13. Vernis à ongles selon l'une quelconque des revendications précédentes, **caractérisée en ce qu'**il comprend au moins un polymère filmogène additionnel

14. Vernis à ongles selon la revendication 13, **caractérisée en ce que** le polymère filmogène additionnel est choisi parmi les polymères cellulosiques tels que la nitrocellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.

15. Vernis à ongles selon la revendication 13 ou 14, **caractérisée en ce que** le polymère filmogène additionnel est présent en une teneur allant de 0,1 % à 60 % en poids, par rapport au poids total de la composition, de préférence allant de 2 % à 40 % en poids, et mieux de 5 % à 25 % en poids.

16. Vernis à ongles selon l'une quelconque des revendications précédentes, **caractérisée en ce qu'**il comprend au moins un agent auxiliaire de filmification et/ou un agent épaississant.

17. Vernis à ongles selon l'une quelconque des revendications précédentes, **caractérisée en ce qu'**il comprend au moins une matière colorante.

18. Vernis à ongles selon la revendication 17, **caractérisé en ce que** la matière colorante est présente en une teneur allant de 0,01 % à 50 % en poids, par rapport au poids de la composition, de préférence de 0,01 % à 30 % en poids.

19. Procédé cosmétique de maquillage ou de soin non thérapeutique des ongles comprenant l'application sur les ongles d'au moins une couche d'une composition de vernis à ongles selon l'une des revendications 1 à 18.

20. Utilisation d'une composition de vernis à ongles comprenant au moins un ester de cellulose et un milieu solvant organique comprenant de l'acétate d'éthyle et de l'acétate de butyle en un ratio acétate d'éthyle/acétate de butyle allant de 87/25 à 100/1, l'acétate d'éthyle étant présent en une teneur supérieure ou égale à 25% en poids par rapport au poids total de la composition et ledit vernis comprenant moins de 5% en poids d'acétone, pour obtenir, après dépôt sur l'ongle, un film qui sèche rapidement, qui est brillant, de bonne tenue et résistant à l'eau.
